# EUROPEAN PATENT APPLICATION

(11) **EP 2 823 851 A2**
(43) Date of publication of application: **14.01.2015**
(21) Application number: 14151900.9
(22) Date of filing: 21.01.2014
(51) Int. Cl.: A61M 39/10, F16L 27/00

(54) **Rotatable connector for medical use**

(30) Priority: 19.06.2013 TW 102211468
(71) Applicant: Mouldex Co., Ltd., Hsinchu Hsien (TW)
(72) Inventor: Chiu, Tzu-Feng, Hsinchu Hsien (TW)
(74) Representative: Gee, Steven William

(57) **Abstract**

A rotatable connector for medical use is provided, applicable to connecting infusion tubings or serving as part of infusion tubing, including a first tube (1), a second tube (2), a casing cover (3), and an elastic sealing member (4); the first tube having a coupling end, the coupling end having an outer wall disposed with a stopper; the second tube having a docking member; the casing cover having one end sheathing outside of stopper and the other end fixed to docking member; a housing space being formed by inner wall of casing cover and outer wall of coupling end; the elastic sealing member being disposed inside housing space, tightly adhered to outer wall of coupling end as well as diving housing space into two disconnected spaces. As such, when applied to infusion tubings, the first tube of the connector of the present invention can rotate to resolve a blockage and free of leakage.

## Description

### CROSS-REFERENCE TO RELATED APPLICATION

The present application is based on, and claims priority from, Taiwan Patent Application No. 102211468 filed on June 19, 2013, the disclosure of which is hereby incorporated by reference herein in its entirety.

### TECHNICAL FIELD

The technical field generally relates to a medical connector technical field, and in particular to a rotatable connector so that the infusion tubings connected to two ends of connector are able to rotate coaxially in opposite directions.

### BACKGROUND

Infusion tubings are a medical supply widely used for liquid infusion, such as, liquid medicine, blood, saline, nutrient, etc., between the medical devices, bottle and patient. Different infusion tubings are connected through various kinds of standard connectors, such as, Luer Connector, to become a complete liquid transport path. Special caution must be taken so that the infusion tubing would not get twined or twisted to cause blockage or disconnected. At present, the infusion tubing is manufactured to have suitable length and/or suitable tube wall thickness to reduce the possibility of blockage caused by the twist and to facilitate the medical staff to relieve the condition.

However, in the medical treatment, it is sometimes difficult to complete control a patient, who may be a human or an animal. During the infusion, the patient may move voluntarily or involuntarily, which may accidentally cause the infusion tubings to get twined or twisted and become blocked. If this condition cannot be easily lifted, the infusion tubing must be re-administered or a new infusion tubing must be connected, which puts extra stress to both the patient and the medical staff. Therefore, it is desirable to devise a rotatable mechanism so that the blocked infusion tubing can be rotated independently without affecting other infusion tubings to lift the blockage condition. However, the desirable mechanism must be carefully designed to prevent the potential leakage problem in the rotation, and thus is an essential issue must be solved.

### SUMMARY

The primary object of the present invention to provide a rotatable connector for medical use, applicable to the connection of two infusion tubings or directly serving as part of infusion tubing. The present invention enables the two connected infusion tubings to rotate coaxially in opposite direction so that when the infusion tubings are blocked due to twisting, the blockage condition can be quickly lifted without re-administering the injection to the patient, adjusting medical instruments or positions of the bottle.

Another object of the present invention is to provide a connector for medical use, simple in structure, rotatable, leak-proof, easy to manufacture and low in cost. When applied to animals or uncontrollable patients during medical infusion, the medical staff can easily and quickly lift the infusion tubing blockage condition.

To achieve the above objects, the present invention provides a rotatable connector, including a first tube, a second tube, a casing cover, and an elastic sealing member; the first tube having a coupling end, the coupling end having an outer wall disposed with a stopper; the second tube having a docking member; the casing cover being a hollow shell, with one end sheathing the outside of the stopper and remaining rotatable, and the other end fixed to the docking member; housing space being formed by inner wall of the casing cover, outer wall of the coupling end and the docking member; the elastic sealing member being disposed inside the housing space and including an inner-ring sealing wall and an outer-ring elastic wall, the inner-ring sealing wall and an outer-ring elastic wall being connected; the inner-ring sealing wall being tightly adhered to the outer wall of the coupling end and the outer-ring elastic wall pushing against the inner wall of the housing space to divide the housing space into two disconnected spaces. As such, during infusion at atmospheric pressure, the first tube of the connector of the present invention can be rotated and free of leakage.

To facilitate easy rotation, the invention also includes a lower friction design. The casing cover is sheathed on the outside of the stopper. The present invention disposes a plurality of bumps on the tube tall of the stopper along the diameter direction. When assembled, the bumps contact the inner wall of the casing cover to reduce the contact area and further reduce the friction in rotation.

The foregoing will become better understood from a careful reading of a detailed description provided herein below with appropriate reference to the accompanying drawings.

### BRIEF DESCRIPTION OF THE DRAWINGS

The embodiments can be understood in more detail by reading the subsequent detailed description in conjunction with the examples and references made to the accompanying drawings, wherein:
FIG. 1 shows a schematic view of the first embodiment of the present invention;
FIG. 2 shows a dissected view of the first embodiment of the present invention;
FIG. 3 shows a dissected view of the first embodiment of the present invention from a different angle;
FIG. 4 shows a cross-sectional view of the first embodiment of the present invention;
FIG. 5 shows the first embodiment of the present invention in actual application, wherein the bottle and the syringe shown in reduced size;
FIG. 6 shows a cross-sectional view of the second embodiment of the present invention; and
FIG. 7 shows a schematic view of the third embodiment of the present invention.

### DETAILED DESCRIPTION OF THE DISCLOSED EMBODIMENTS

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

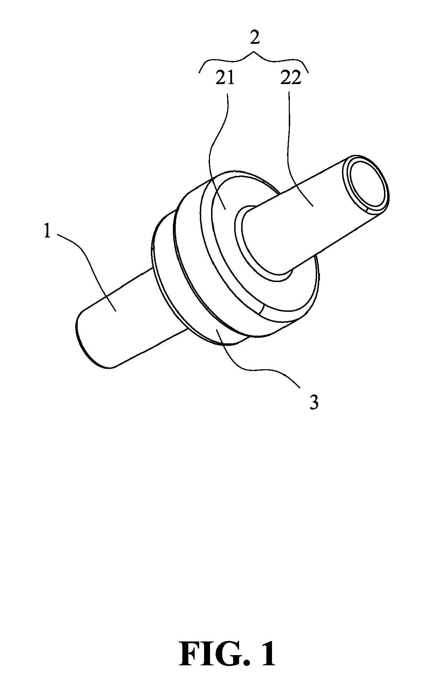

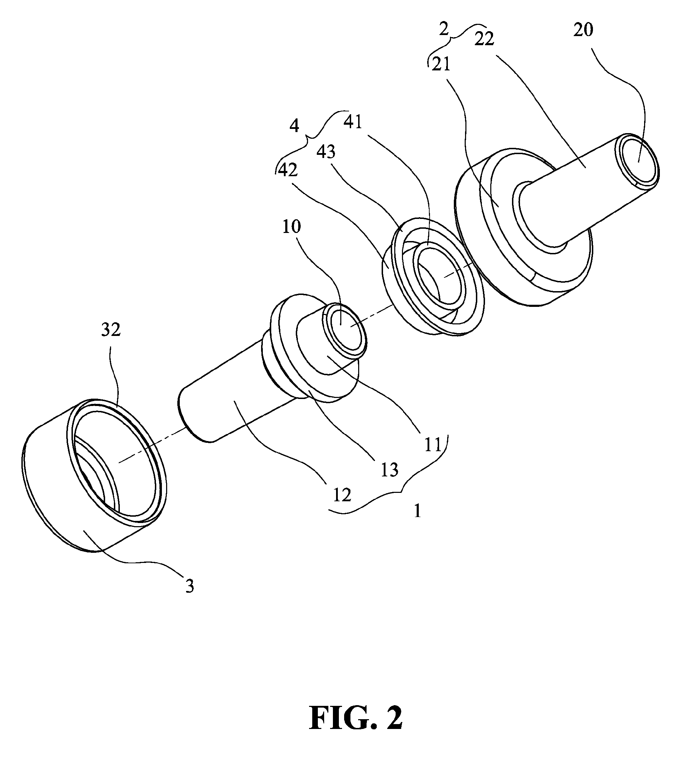

FIG. 1 and FIG. 2 show a schematic view and a dissected view of the present invention according to an exemplary embodiment. In the instant embodiment, a medical connector includes a first tube 1, a second tube 2, a casing cover 3, and an elastic sealing member 4. Through engaging with the casing cover 3, the second tube 2 holds a coupling end 11 of the first tube 1 between the second tube 2 and the casing cover 3. The elastic sealing member 4 is for a tight fitting with the coupling end 11. The assembled state must achieve complete leak-proof during infusion or when the first tube 1 rotates.

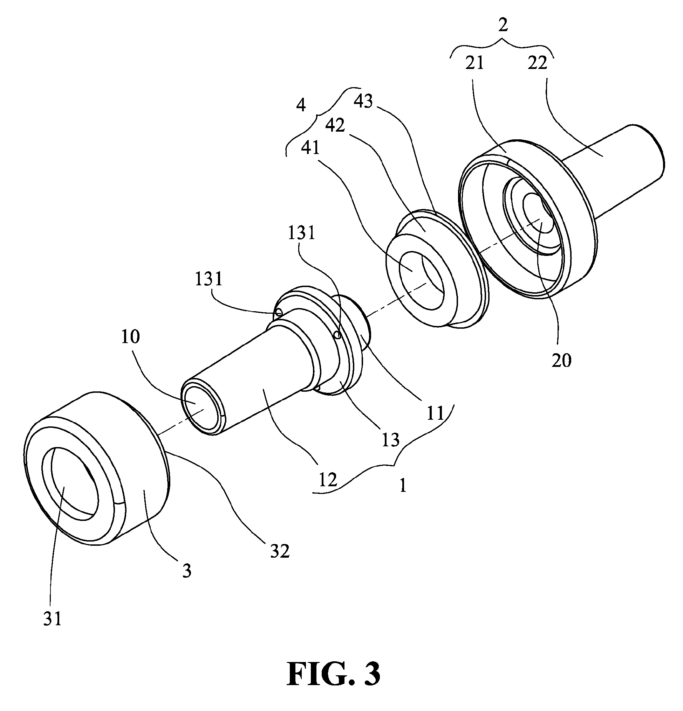

The following describes the structure of each component in details. The first tube 1 is a hollow tube body, having a central path 10 for liquid passage, with one end as a coupling end 11 and the other end as a tube connector 12. A stopper 13 is disposed at a location near the coupling end 11. The stopper 13 is a ring-shape plate protruding from the tube wall. The tube wall of the stopper 13 in the diameter direction facing the tube connector 12 is disposed with a plurality of bumps 131 (as shown in FIG. 3). The tube connector 12 is of a standard specification. In the instant embodiment, the tube connector is a Luer slip connector, and a male connector. However, the tube connector is not restricted to the present embodiment, and a female connector or a Luer lock connector can also be used.

The second tube 2 is a hollow tube body, having a central path 20 for liquid passage, with one end as a docking member 21 and the other end as a tube connector 22. The docking member 21 has a disc shape for engaging the casing cover 3. The tube connector 22 is of a standard specification. In the instant embodiment, the tube connector is a Luer slip connector, and a male connector. However, the tube connector is not restricted to the present embodiment, and a female connector or a Luer lock connector can also be used.

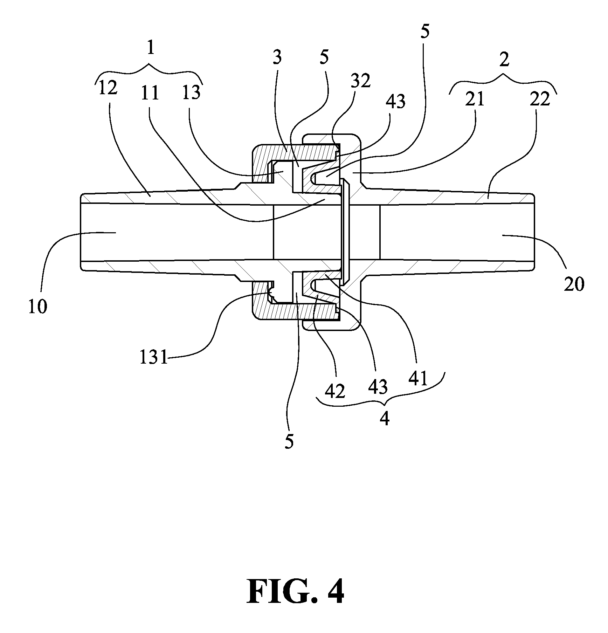

The casing cover 3 is a hollow circular tube cover, with one end having a small diameter hole 31 for the tube connector 12 of the first tube 1 to pass. The stopper 13 is restricted to remain inside the casing cover 3. The other end of the casing cover 3 is an opening end 32 with a larger diameter for engaging the docking member 22 of the second tube 2. For assembly, the opening end 32 of the casing cover 3 is fixed to the inner wall of the docking member 22 of the second tube 2 through ultrasonic welding. When assembled, the inner wall of the casing cover 3, a housing space 5 (as shown in FIG. 4) is formed by the outer wall of the coupling end 11 and the docking member 21. The elastic sealing member 4 is disposed inside the housing space 5. To reduce the rotational friction, the hole 31 of the casing cover 3 is slightly larger than the outer diameter of the tube connector 12 and the size of the inner wall is also slightly larger than the outer diameter of the stopper 13. The tube wall of the stopper 13 in the diameter direction uses the bumps 131 to contact the inner wall of the casing cover 3 so as to reduce the contact area.

The elastic sealing member 4 is made of soft elastic material. In the instant embodiment, the material is silicone, which has good elastic property and sealing effect. The elastic sealing member 4 includes an inner-ring sealing wall 41 and an outer-ring elastic wall 42. The inner-ring sealing wall 41 and the outer-ring elastic wall 42 are connected. The outer-ring elastic wall 42 has a conic shape and is connected to the inner-ring sealing wall 41. The inner-ring sealing wall 41 is to tightly adhere to the outer wall of the coupling end 11 when assembled to achieve leak-proof result. However, when an external force exceeding a default value is applied, the coupling end 11 can be rotated while the inner-ring sealing wall 41 remains good leak-proof effect. When assembled, the outer-ring elastic wall 42 pushes against the inner wall of the housing space 5. In addition to maintain the inner-ring sealing wall 41 to remain in a better sealing result, the outer-ring elastic wall 42 also divide the housing space 5 into two disconnected spaces for preventing the liquid from flowing out of the connection of casing cover 3 and the first tube 1.

For easy assembly and tight holding, the elastic sealing member 4 further includes a sealing lip 43, disposed at the edge of the circumference of the outer-ring elastic wall 42. When assembled, the sealing lip 43 is located tightly between the connection of the opening end 32 and the docking member 22 to fix the location of the elastic sealing member 4 as well as achieve good leak-proof result. In the instant embodiment, the outer-ring elastic wall 42 will not be tightly adhered to the inner wall of the casing cover 3 or inner wall of the docking member 21. That is, a gap exists between the partial outer wall and the inner wall of the casing cover 3 so that the inner-ring sealing wall 41 is hanged in an elastic manner. As such, the good sealing result is achieved when assembled. In the instant embodiment, the second tube 2, the casing cover 3 and the elastic sealing member 4 are fixed together and the three moves in a linkage manner.

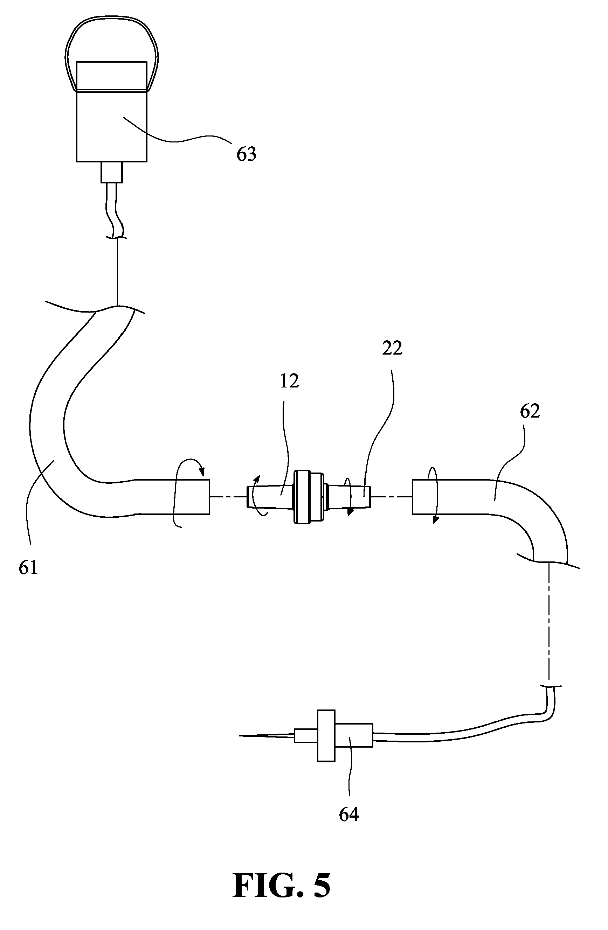

The following describes the actual application of the present invention. As shown in FIG. 5, when the medical connector of the present invention is in actual application, the two ends will be connected to an infusion tubing 61, 62 respectively through the tube connectors 12, 22. The infusion tubing 61 is connected to a bottle 63 and the infusion tubing 62 is connected to a syringe 64. The syringe 64 is to injecting to the patient. If one of the infusion tubings is blocked due to twist, the blockage can be resolved easily and rapidly by rotating one of the infusion tubings connected to the connector of the present invention.

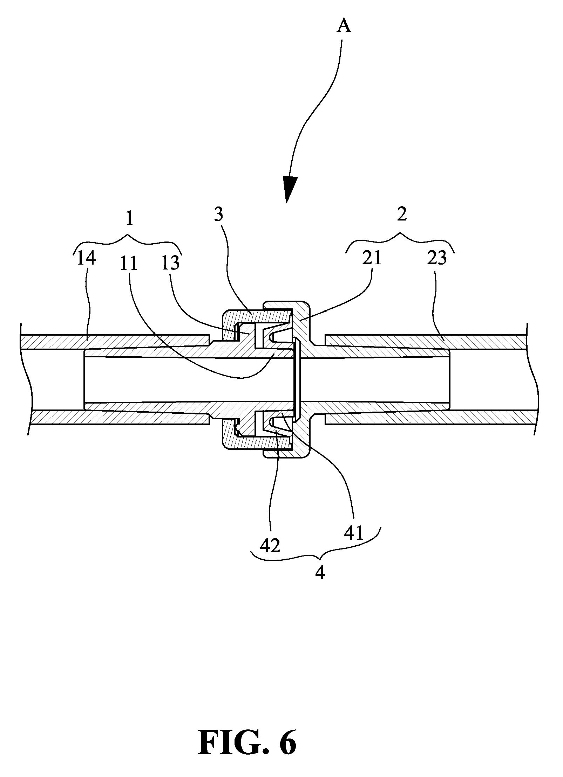

FIG. 6 shows a cross-sectional view of the second embodiment of the present invention. In the instant embodiment, the connector is directly installed an infusion tubing A. The connector still includes a first tube 1, a second tube 2, a casing cover 3 and an elastic sealing member 4. The difference is that the first tube 1 is directly engaged to a tube element 14 of the infusion tubing at the end away from the stopper 11, and the second tube 2 is directly engaged to a tube element 23 of the infusion tubing at the end away from the docking member 12. In other words, the connector is part of the infusion tubing A, and is located at the middle segment. The advantage of this design is that the blockage can be resolved by rotating the first tube 1 or the second tube 2 relatively. The user no longer uses a single connector to connect two infusion tubings. The above description shows that the connector of the present invention is widely applicable to infusion tubings, but is not restricted to the embodiments. The connector can also be disposed at one end of the infusion tubing for connecting another infusion tubing.

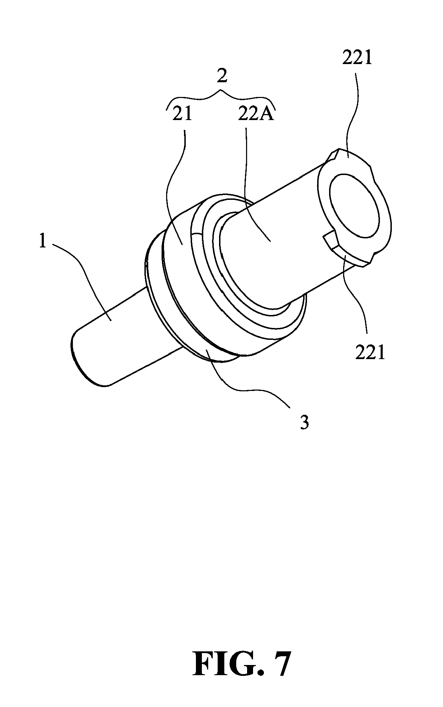

FIG. 7 shows a schematic view of the third embodiment of the present invention. In the instant embodiment, the connector still includes a first tube 1, a second tube 2, a casing cover 3 and an elastic sealing member 4. The difference is that the tube connector 22A of the second tube 2 is a female Luer connector, and the outer wall of the tube connector 22A is disposed with lug 221 so that the tube connector 22A can also serve as a Luer lock connector. As shown, the tube connectors at the two ends of the connector of the present invention are not necessarily of the same type as long as both meet the standard specification.

It will be apparent to those skilled in the art that various modifications and variations can be made to the disclosed embodiments. It is intended that the specification and examples be considered as exemplary only, with a true scope of the disclosure being indicated by the following claims and their equivalents.

## Claims

1. A rotatable connector for medical use, comprising: a first tube, a second tube, a casing cover and an elastic sealing member; wherein:
the first tube having a coupling end, the coupling end having an outer wall disposed with a stopper;
the second tube having a docking member;
the casing cover with one end sheathing outside of the stopper and remaining rotatable, and the other end fixed to the docking member; a housing space being formed by inner wall of the casing cover, outer wall of the coupling end and the docking member;
the elastic sealing member being disposed inside the housing space and comprising an inner-ring sealing wall and an outer-ring elastic wall, the inner-ring sealing wall and an outer-ring elastic wall being connected; the inner-ring sealing wall being tightly adhered to the outer wall of the coupling end and the outer-ring elastic wall pushing against the inner wall of the housing space to divide the housing space into two disconnected spaces.

2. The rotatable connector for medical use as claimed in claim 1, wherein the outer-ring elastic wall has a conic shape and is connected to the inner-ring sealing wall; a gap exists between the partial outer wall and the inner wall of the casing cover so that the inner-ring sealing wall is hanged in an elastic manner.

3. The rotatable connector for medical use as claimed in claim 1, wherein the elastic sealing member further comprises a sealing lip, disposed at the edge of a circumference of the outer-ring elastic wall; the sealing lip is located tightly between the connection of the opening end and the docking member when assembled.

4. The rotatable connector for medical use as claimed in claim 1, wherein the first tube 1 is a hollow tube body, the other end away from the coupling end is a tube connector; the stopper protrudes from the tube wall at the coupling end.

5. The rotatable connector for medical use as claimed in claim 4, wherein the tube wall of the stopper in the diameter direction facing the tube connector is disposed with a plurality of bumps for contacting the inner wall of the casing cover to reduce friction.

6. The rotatable connector for medical use as claimed in claim 1, wherein the other end of the first tube away from the coupling end is a tube element connected to an infusion tubing.

7. The rotatable connector for medical use as claimed in claim 1, wherein the second tube is a hollow tube element and the other end of the second tube away from the docking member is a tube connector.

8. The rotatable connector for medical use as claimed in claim 1, wherein the second tube is a hollow tube element and the other end of the second tube away from the docking member is a tube element connected to an infusion tubing.

9. The rotatable connector for medical use as claimed in claim 1, wherein the casing cover and the docking member are fixed together by ultrasonic welding.

10. The rotatable connector for medical use as claimed in claim 1, wherein the rotatable connector is installed to an infusion tubing.

11. The rotatable connector for medical use as claimed in claim 1, wherein the rotatable connector is installed to an end of an infusion tubing.
